# EUROPEAN PATENT APPLICATION

(11) **EP 4 744 701 A1**
(43) Date of publication of application: **20.05.2026**
(21) Application number: 24839387.8
(22) Date of filing: 10.06.2024
(51) Int. Cl.: A61M 16/00

(54) **EXHALATION ASSISTANCE DEVICE AND ARTIFICIAL RESPIRATION DEVICE**

(30) Priority: 12.07.2023 JP 2023114307
(71) Applicant: Tohoku University, Sendai-shi, Miyagi 980-8577 (JP)
(72) Inventor: TAKAHASHI, Kazuhiro, Sendai-shi, Miyagi 980-8577 (JP); YAMAUCHI, Masanori, Sendai-shi, Miyagi 980-8577 (JP); TOYAMA, Hiroaki, Sendai-shi, Miyagi 980-8577 (JP); ISHIKAWA, Takuji, Sendai-shi, Miyagi 980-8577 (JP); KIKUCHI, Kenji, Sendai-shi, Miyagi 980-8577 (JP); ARATA, Yutaro, Sendai-shi, Miyagi 980-8577 (JP); EJIMA, Yutaka, Sendai-shi, Miyagi 980-8577 (JP)
(74) Representative: Plougmann Vingtoft a/s
(86) International application number: PCT/JP2024/020990
(87) International publication number: WO 2025/013490

(57) **Abstract**

[Problem] To provide an exhalation assistance device and artificial respiration device that can utilize the functions of an artificial respiration device familiar to medical professionals, are easy to control, and can be introduced at a low cost.

[Solution] An exhalation assistance device is used by being attached to an artificial respiration device 1 having a patient-side flow path provided such that one end side can be attached to a patient 51, and an inhalation-side flow path and exhalation-side flow path communicating with the other end of the patient-side flow path. A biasing means 11 is provided so as to be attachable to the patient-side flow path and configured to, when attached to the patient-side flow path, allow the inspiratory air supplied to the patient 51 to pass through while biasing expiratory air from the patient 51 in a discharge direction. A sensor 12 is provided such that the pressure in each flow path and/or the flow rate of the inspiratory air and expiratory air of the patient 51 can be measured. A control unit 13 is connected to the biasing means 11 and the sensor 12 and is provided to be capable of drive controlling the biasing means 11 during exhalation based on measurement data from the sensor 12.

## Description

### Field of the Invention

The present invention relates to an exhalation assistance device and an artificial respiration device.

### Description of Related Art

In patients with chronic obstructive pulmonary disease (COPD), such as emphysema or chronic bronchitis, airway narrowing and loss of lung elastic recoil make exhalation difficult and prolong expiratory time. As a result, COPD often leads to respiratory failure. Additionally, even COPD patients may undergo surgery under general anesthesia.

Patients with respiratory failure or those whose spontaneous breathing has ceased due to general anesthesia require support from artificial respiration. During artificial respiration, the volume and speed of inspiratory air delivered to the patient's lungs can be adjusted during inhalation. However, during exhalation, the lungs depend on their elastic recoil to return to their pre-inspiratory volume, making it difficult to regulate expiratory flow. For this reason, when performing artificial ventilation on a COPD patient with reduced lung elasticity, exhalation takes a prolonged time, and the transition to inhalation occurs before sufficient exhalation is achieved, leading to lung overexpansion and a decrease in ventilation volume per unit time (minute ventilation volume), which poses a risk of causing hypoxemia or hypercapnia.

Conventionally, examples of devices that assist with exhalation include a forced inspiration and exhalation device (see Patent Literature 1) including a patient interface unit configured to allow a negative pressure airflow to pass through and deliver a positive pressure airflow from a medical mechanical ventilator, and a suction unit for generating the negative pressure airflow passing through the patient interface unit. Another example is an artificial respiration device (see Patent Literature 2) which supplies inspiratory air to the patient and suctions expiratory air from the patient, the device including an inner bellows, an outer bellows provided to cover the inner bellows, an exhalation conduit connected to a first space inside the inner bellows, and an inhalation conduit connected to a second space between the inner and outer bellows.

Additionally, a commercially available device, "EVONE" (registered trademark, manufactured by Ventinova Medical), which utilizes the Venturi effect to assist with both inhalation and exhalation of the patient, is already in use during surgery or intensive care (see Non-Patent Literature 1).

### Citation List

### Patent Literature

Patent Literature 1: JP 2009-509610 A
Patent Literature 2: JP 2002-200168 A

### Non-Patent Literature

Non-Patent Literature 1: Ventinova Medical, "evone," [online], [retrieved on June 14, 2023], Internet <URL: https://www.ventinovamedical.com/evone/>

### Summary of the Invention

### [Problems to be solved by the invention]

In the forced inspiration and exhalation device described in Patent Literature 1, using a separate flow path from the one that supplies inspiratory air (positive pressure airflow) to the patient from a medical mechanical ventilator via a patient interface unit, the suction unit generates suction force to cause forced exhalation in the patient, and multiple check valves (gates) are used to prevent inspiratory and expiratory air from mixing in each other's flow paths. For this reason, there was an issue that the control of medical mechanical ventilators, suction units, and various check valves becomes complex. Additionally, even when using artificial respiration devices such as ventilators or anesthesia machines, which medical professionals are accustomed to as medical mechanical ventilators, it is necessary to separately monitor the exhaust from the suction unit, presenting the issue of having to monitor the patient under a different system than conventional methods.

The artificial respiration device described in Patent Literature 2 performs both the supply of inspiratory air to the patient and the suction of the expiratory air from the patient, and is a standalone device separate from commercially available artificial respiration devices, such as ventilators or anesthesia machines, that medical professionals are accustomed to using. For this reason, there was an issue that the introduction cost is high, and it is necessary to become newly accustomed to its use.

The respiratory assistance device described in Non-Patent Literature 1 is also a standalone device separate from commercially available artificial respiration devices, such as ventilators or anesthesia machines, that medical professionals are accustomed to using. For this reason, there was an issue that the introduction cost is high, and it is necessary to become newly accustomed to its use.

The present invention addresses these issues and aims to provide an exhalation assistance device and an artificial respiration device that can utilize the functions of an artificial respiration device familiar to medical professionals, are easy to control, and can be introduced at a low cost.

### [Means for Solving the Problem]

To achieve the above objective, an exhalation assistance device according to the present invention is an exhalation assistance device used by being attached to an artificial respiration device to assist the discharge of expiratory air from a patient, the artificial respiration device having a patient-side flow path provided such that one end side can be attached to the patient, and an inhalation-side flow path and an exhalation-side flow path communicating with the other end of the patient-side flow path, the artificial respiration device configured to supply inspiratory air to the patient from the inhalation-side flow path through the patient-side flow path to inflate the patient's lungs during inhalation, and to discharge expiratory air from the patient from the patient-side flow path through the exhalation-side flow path during exhalation, the exhalation assistance device including: a biasing means provided to be attachable to the patient-side flow path and configured to, when attached to the patient-side flow path, allow the inspiratory air supplied to the patient to pass through while biasing expiratory air from the patient in a discharge direction; a sensor provided to be capable of measuring a pressure in at least one of the patient-side flow path, the inhalation-side flow path, or the exhalation-side flow path, and/or a flow rate of the inspiratory and expiratory air of the patient; and a control unit connected to the biasing means and the sensor and provided to be capable of drive controlling the biasing means during exhalation based on measurement data from the sensor.

The exhalation assistance device according to the present invention is used by being attached to an artificial respiration device, such as a ventilator or anesthesia machine, familiar to medical professionals. The biasing means is attached to the patient-side flow path, through which both inspiratory and expiratory air pass, and is configured to allow inspiratory air supplied to the patient to pass through while biasing expiratory air from the patient in the discharge direction. Therefore, the exhalation assistance device does not require check valves, has a relatively simple structure, and is easy to control. Additionally, by synchronizing with the artificial respiration device, the exhalation assistance device can assist with the discharge of the expiratory air from the patient, enabling appropriate ventilation for patients with respiratory failure or COPD, for example, thereby preventing hypoxemia or hypercapnia.

Since the exhalation assistance device according to the present invention can be used by being attached to an artificial respiration device, such as a ventilator or anesthesia machine, the functions of the artificial respiration device, such as mechanical ventilation such as pressure-controlled ventilation, volume-controlled ventilation, or pressure support ventilation, manual ventilation using a breathing bag, measurement of ventilation volume, and recirculation of anesthesia gases, can be used as they are. Therefore, medical professionals do not need to become accustomed to a new artificial respiration device. Moreover, since the exhalation assistance device can be retrofitted to the artificial respiration device immediately, it is suitable for emergency use. Additionally, compared to purchasing a new device to control the entire respiratory process, the exhalation assistance device can be introduced at a lower cost.

In the exhalation assistance device according to the present invention, it is preferable that all flow paths, from one end of the patient-side flow path through the biasing means to the ends of the inhalation-side and exhalation-side flow paths on the artificial respiration device side, are configured to be airtight to efficiently assist with the discharge of expiratory air and supply inspiratory air. In the exhalation assistance device according to the present invention, the biasing means may include any means capable of biasing the expiratory air from the patient in the discharge direction, such as, for example, a centrifugal pump. The biasing means, for example, may be configured such that a centrifugal pump or the like is attached to the patient-side flow path with a gap provided to allow the passage of inspiratory air supplied to the patient. It is also preferable that the biasing means is provided to be airtight except at the portion communicating with the patient-side flow path. Additionally, in the exhalation assistance device according to the present invention, it is preferable that the control unit is configured to be capable of controlling, for example, the biasing force or operating time of the biasing means.

In the exhalation assistance device according to the present invention, the sensor may include a pressure sensor configured to measure the pressure in at least one of the patient-side flow path, inhalation-side flow path, or exhalation-side flow path. Since the patient-side flow path, inhalation-side flow path, and exhalation-side flow path are interconnected and maintain a constant internal pressure, the pressure sensor may be provided in any of these flow paths. The exhalation assistance device may also include a flow sensor configured to measure the flow rate of the inspiratory and expiratory air of the patient. To avoid the influence of bias flow circulating through the main body of the artificial respiration device, the exhalation-side flow path, and the inhalation-side flow path, the flow sensor is preferably provided in the patient-side flow path. The flow sensor may be positioned either downstream or upstream of the biasing means along the discharge direction of expiratory air of the patient. In these cases, the control unit can drive control the biasing means during exhalation in synchronization with the breathing of the patient.

The exhalation assistance device according to the present invention may be configured to allow the biasing means or sensor to be retrofitted to each tube constituting the patient-side flow path, inhalation-side flow path, or exhalation-side flow path, which are provided to be connectable to an existing artificial respiration device, such as a ventilator or anesthesia machine. Alternatively, it may be configured to replace them entirely as an integrated unit, including each tube. In this case, the exhalation assistance device according to the present invention preferably includes, for example, a patient-side tube provided to be capable of constituting the patient-side flow path, to which the biasing means is attached; a branching joint attached to the other end of the patient-side tube so that the patient-side flow path branches into the inhalation-side flow path and the exhalation-side flow path on the other end side of the patient-side tube; an inhalation-side tube connected to the branching joint to constitute the inhalation-side flow path; and an exhalation-side tube connected to the branching joint to constitute the exhalation-side flow path.

In the case of an integrated replacement configuration, the exhalation assistance device according to the present invention preferably includes an intubation tube provided to be attachable to one end of the patient-side tube to communicate with the patient-side flow path, and the intubation tube is configured to be capable of tracheal intubation in the patient to supply inspiratory air to the patient and discharge the expiratory air from the patient. Moreover, the intubation tube preferably has an outer diameter of 7 mm or less. Since the exhalation assistance device according to the present invention can assist with the discharge of expiratory air through the biasing means, sufficient respiration can be ensured even with a thin intubation tube with an outer diameter of 7 mm or less. This can reduce throat pain in the patient during intubation and facilitate intubation in patients with upper airway stenosis. Additionally, it can assist non-invasive artificial respiration without intubation of the patient. The intubation tube may also be configured to be retrofitted to the tip of the tube constituting the patient-side flow path of the artificial respiration device.

The artificial respiration device according to the present invention includes the exhalation assistance device according to the present invention. The artificial respiration device according to the present invention can assist with the discharge of the expiratory air from the patient during artificial respiration through the exhalation assistance device according to the present invention. The artificial respiration device according to the present invention may be any device capable of performing artificial respiration, such as a ventilator or anesthesia machine.

### [Advantageous Effects of Invention]

According to the present invention, it is possible to provide an exhalation assistance device and an artificial respiration device that can utilize the functions of an artificial respiration device familiar to medical professionals, are easy to control, and can be introduced at a low cost.

### Brief Description of the Drawings

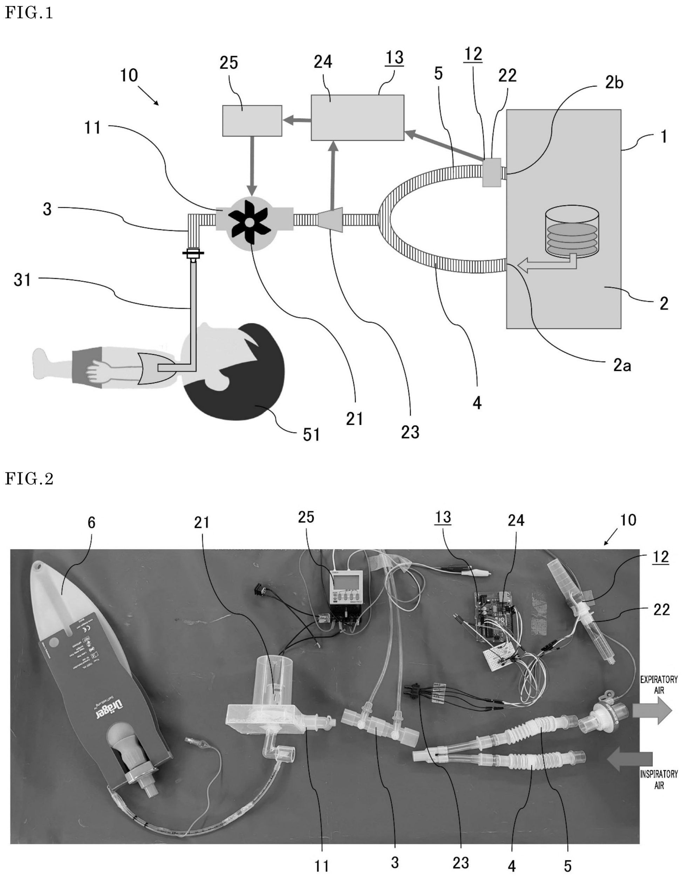

[FIG. 1] is an overall configuration diagram showing the usage state of an exhalation assistance device according to an embodiment of the present invention.
[FIG. 2] is a plan view showing the exhalation assistance device and test lung used in a model experiment.

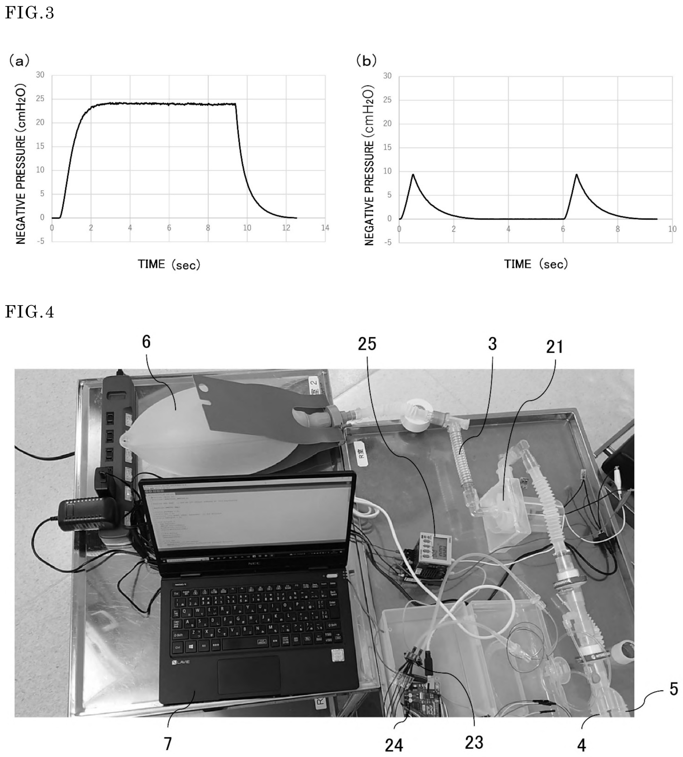

[FIG. 3] shows graphs showing the negative pressure fluctuations of the exhalation assistance device according to an embodiment of the present invention when (a) the centrifugal pump is operated for a sufficiently long time (approximately 9 seconds), and (b) the centrifugal pump is operated for 0.5 seconds.
[FIG. 4] is a perspective view showing the model experiment using the exhalation assistance device shown in FIG. 1.

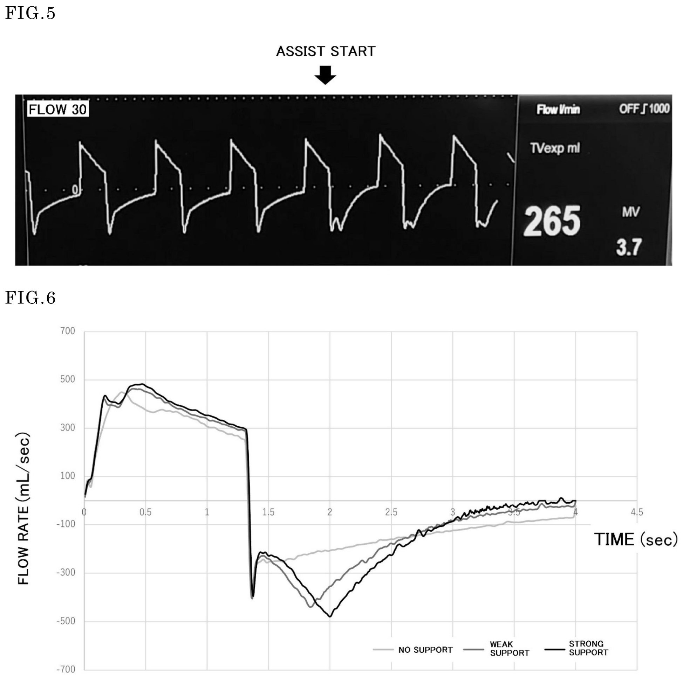

[FIG. 5] is a graph showing the fluctuations in flow rate during respiration displayed on the monitor of an artificial respiration device in the model experiment shown in FIG. 4.
[FIG. 6] is a graph superimposing flow rate fluctuation data for one respiratory cycle in a model experiment using the exhalation assistance device shown in FIG. 1 with a modified test lung from FIG. 4, comparing cases when the exhalation assistance device shown in FIG. 1 is not operated (no support), when operated with a centrifugal pump operating time of 0.4 seconds (weak support), and when operated with a centrifugal pump operating time of 0.5 seconds (strong support).

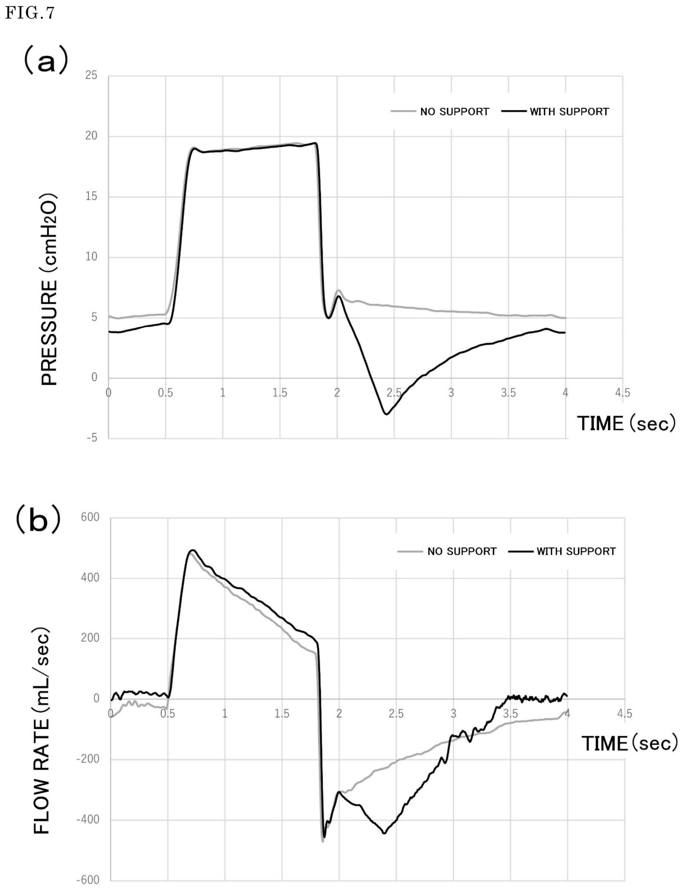

[FIG. 7] shows graphs superimposing (a) pressure fluctuation data and (b) flow rate fluctuation data for one respiratory cycle in an animal experiment using pigs with the exhalation assistance device shown in FIG. 1, comparing cases when the exhalation assistance device shown in FIG. 1 is not operated (no support) and when operated (with support).

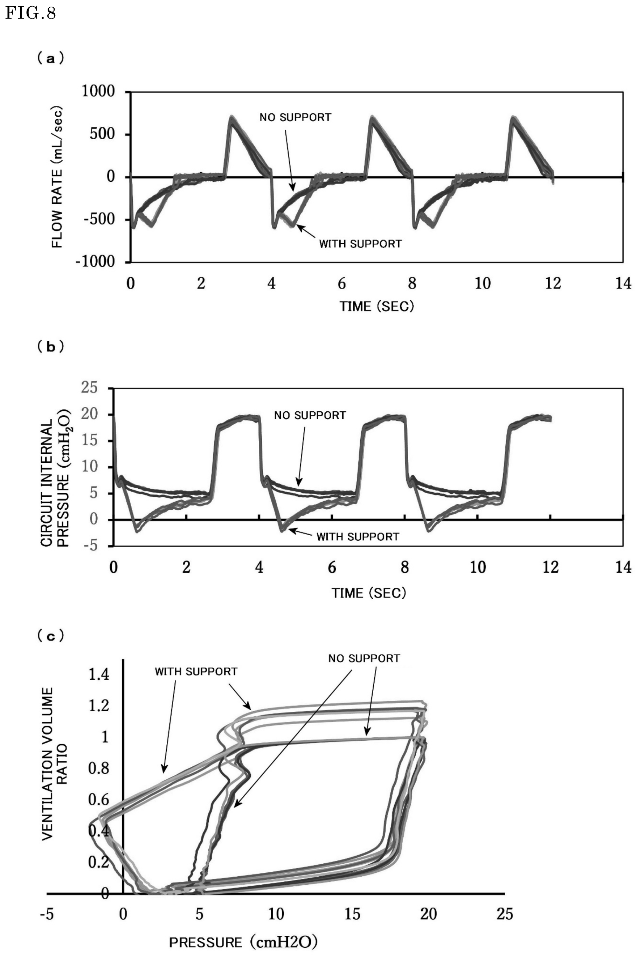

[FIG. 8] shows graphs superimposing (a) flow rate, (b) circuit internal pressure fluctuation data, and (c) a curve (PV loop) connecting the relationship between circuit internal pressure and ventilation volume ratio for three consecutive respiratory cycles in an animal experiment using six pigs with the exhalation assistance device shown in FIG. 1, comparing cases when the exhalation assistance device shown in FIG. 1 is not operated (no support) and when operated (with support).

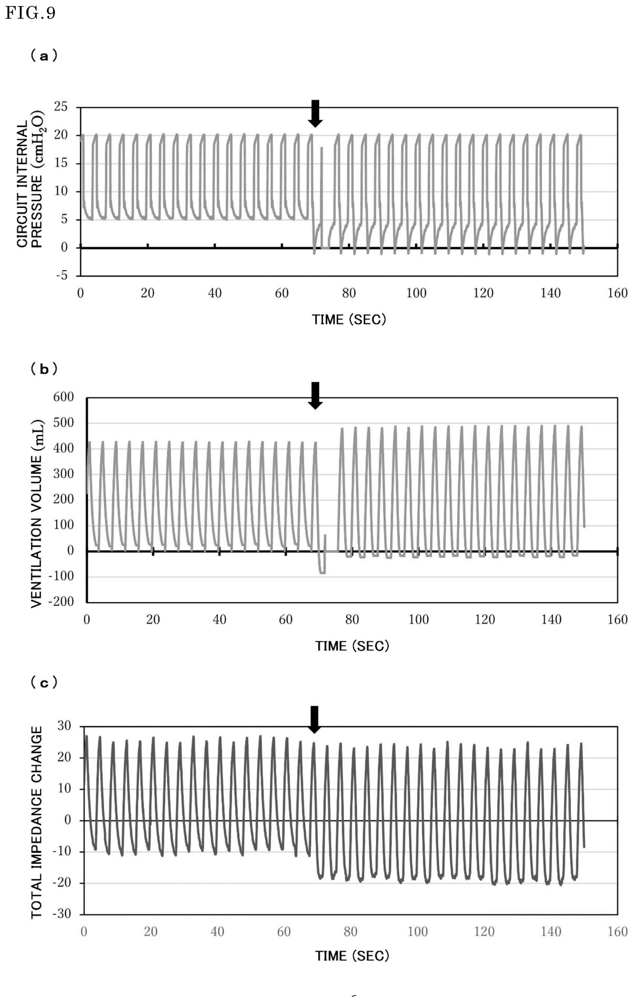

[FIG. 9] shows graphs showing the temporal changes in (a) circuit internal pressure, (b) ventilation volume, and (c) total impedance change before and after operating the exhalation assistance device in an animal experiment using pigs with the exhalation assistance device shown in FIG. 1.

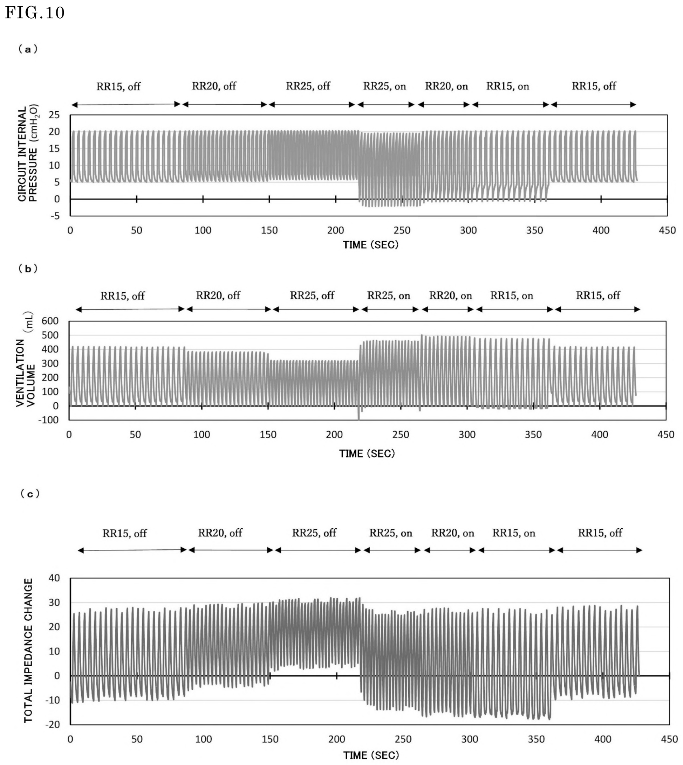

[FIG. 10] shows graphs showing the temporal changes in (a) circuit internal pressure, (b) ventilation volume, and (c) total impedance change when varying the respiratory rate (RR) and the on/off state of the exhalation assistance device in an animal experiment using pigs with the exhalation assistance device shown in FIG. 1.

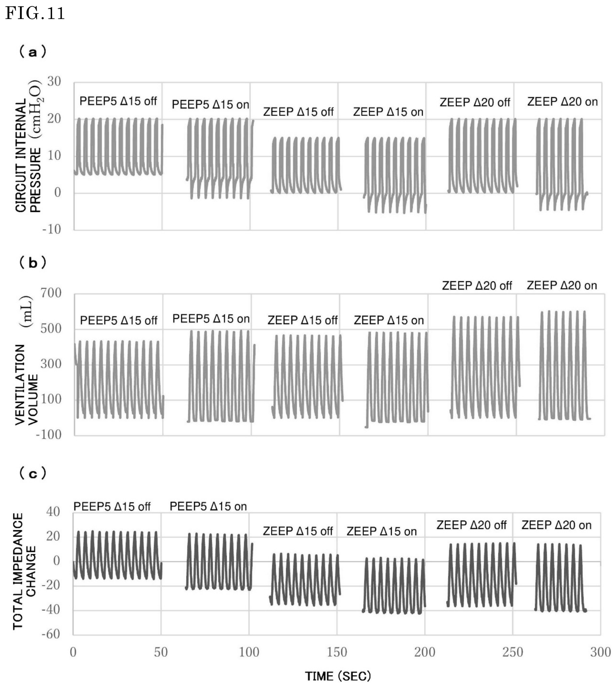

[FIG. 11] shows graphs showing the temporal changes in (a) circuit internal pressure, (b) ventilation volume, and (c) total impedance change when varying the inspiratory pressure (driving pressure Δ), positive end-expiratory pressure (PEEP), and the on/off state of the exhalation assistance device in an animal experiment using pigs with the exhalation assistance device shown in FIG. 1.

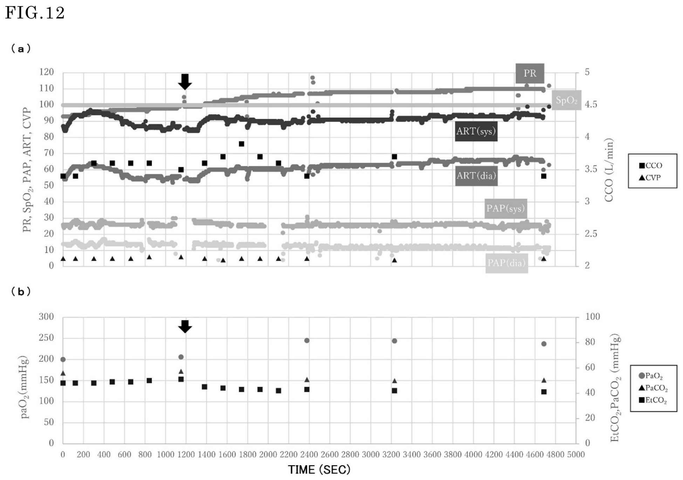

[FIG. 12] shows graphs showing the temporal changes in (a) pulse rate (PR), percutaneous arterial blood oxygen saturation (SpO₂), pulmonary artery pressure (PAP) (systolic: sys, diastolic: dias), arterial pressure (ART) (systolic: sys, diastolic: dias), central venous pressure (CVP), continuous cardiac output (CCO), and (b) arterial blood oxygen partial pressure (PaO₂), end-tidal carbon dioxide partial pressure (EtCO₂), and arterial blood carbon dioxide partial pressure (PaCO₂) before and after operating the exhalation assistance device in an animal experiment using pigs with the exhalation assistance device shown in FIG. 1.

### Detailed Description of the Invention

Hereinafter, embodiments of the present invention will be described with reference to the drawings and other materials.

FIGS. 1 to 12 illustrate the exhalation assistance device according to an embodiment of the present invention.

As shown in FIG. 1, an exhalation assistance device 10 is used by being attached to an artificial respiration device 1, such as a ventilator or anesthesia machine, and includes a biasing means 11, a sensor 12, and a control unit 13.

The artificial respiration device 1 includes a device body 2, a first tube 3, a second tube 4, and a third tube 5. The device body 2 has an inhalation port 2a for supplying inspiratory air to a patient 51 and an exhalation port 2b for collecting expiratory air from the patient 51 to perform artificial respiration on the patient 51. The first tube 3 is provided such that one end can be attached to the patient 51. The second tube 4 is provided such that one end communicates with the other end of the first tube 3, and the other end is connectable to the inhalation port 2a of the device body 2. The third tube 5 is provided such that one end communicates with the other end of the first tube 3, and the other end is connectable to the exhalation port 2b of the device body 2. The artificial respiration device 1 is configured to be airtight across all flow paths through which the inspiratory and expiratory air of the patient 51 passes, from one end of the first tube 3 to the other ends of the second tube 4 and third tube 5 connected to the device body 2.

The artificial respiration device 1 is configured to supply inspiratory air to the patient 51 through the inhalation port 2a, second tube 4, and first tube 3 to inflate the lungs of the patient 51 during inhalation. During exhalation, the artificial respiration device 1 is configured to collect and discharge expiratory air from the patient 51 through the first tube 3 and third tube 5 via the exhalation port 2b. The first tube 3 constitutes the patient-side flow path, the second tube 4 constitutes the inhalation-side flow path, and the third tube 5 constitutes the exhalation-side flow path.

The biasing means 11 includes a centrifugal pump 21 and is provided to be attachable to the first tube 3. The centrifugal pump 21 is arranged such that, when the biasing means 11 is attached to the first tube 3, a gap is provided to allow the inspiratory air supplied to the patient 51 to pass through. The biasing means 11 is configured to, when attached to the first tube 3, allow the inspiratory air supplied to the patient 51 to pass through while biasing the expiratory air from the patient 51 in the discharge direction by the centrifugal pump 21. Additionally, the biasing means 11 is provided to be airtight except at the portion communicating with the first tube 3.

The sensor 12 includes a pressure sensor 22 and a flow sensor 23. The pressure sensor 22 may be attached to any one of the first tube 3, the second tube 4, or the third tube 5 to measure the pressure inside at least one of these tubes, but in the specific example shown in FIG. 1, it is provided to be attachable to the third tube 5. The flow sensor 23 is provided to be attachable to the first tube 3 to measure the flow rate of the inspiratory and expiratory air of the patient 51. In the specific example shown in FIG. 1, the flow sensor 23 is provided to be attachable to the first tube 3 on the downstream side of the biasing means 11 along the discharge direction of the expiratory air of the patient 51.

The control unit 13 includes a microcomputer 24 connected to the pressure sensor 22 and the flow sensor 23, and a relay circuit 25 connected to the microcomputer 24 and the centrifugal pump 21. The microcomputer 24 is configured to receive measurement data from the pressure sensor 22 and the flow sensor 23 and, based on the measurement data, send an operation signal to the relay circuit 25 in synchronization with the timing of the exhalation of the patient 51. The microcomputer 24 is configured to set and adjust the operating time or rotational speed of the centrifugal pump 21 and send the operating time or rotational speed to the relay circuit 25 via the operation signal. The relay circuit 25 is configured to receive the operation signal and supply power to the centrifugal pump 21 in accordance with the operation signal to operate the centrifugal pump 21. Thus, the control unit 13 is configured to be capable of drive controlling the biasing means 11 during exhalation.

The control unit 13 may drive control the biasing means 11 using the measurement data from both the pressure sensor 22 and the flow sensor 23, but may drive control the biasing means 11 using the measurement data from only one of the pressure sensor 22 or the flow sensor 23. Additionally, the control unit 13 may be configured to perform various controls, such as automatically controlling the operating time or rotational speed of the centrifugal pump 21 based on the measurement data from the pressure sensor 22 and the flow sensor 23, or stopping the centrifugal pump 21 when the measurement data from the pressure sensor 22 or the flow sensor 23 indicates an abnormal value.

Next, the operation will be described.

The exhalation assistance device 10 can be used by retrofitting the biasing means 11 and the flow sensor 23 to the first tube 3 and the pressure sensor 22 to the third tube 5, among the tubes provided to be connectable to an existing artificial respiration device 1, such as a ventilator or anesthesia machine, familiar to medical professionals, to assist with the discharge of the expiratory air from the patient 51. The exhalation assistance device 10 is used in a state where the other end of the second tube 4 is connected to the inhalation port 2a of the device body 2 of the artificial respiration device 1, the other end of the third tube 5 is connected to the exhalation port 2b of the device body 2, and one end of the first tube 3 is attached to the mouth or nose of the patient 51.

The exhalation assistance device 10 is configured such that the biasing means 11 is attached to the first tube 3, through which the inspiratory air and expiratory air from the patient 51 passes, to allow the inspiratory air supplied to the patient 51 to pass through while biasing the expiratory air from the patient 51 in the discharge direction. Therefore, the exhalation assistance device 10 does not require check valves, has a relatively simple structure, and is easy to control. Additionally, by synchronizing with the artificial respiration device 1, the device can assist with the discharge of the expiratory air from the patient 51, enabling appropriate ventilation for patients 51 with respiratory failure or COPD, for example, thereby preventing hypoxemia or hypercapnia.

Since the pressure sensor 22 of the exhalation assistance device 10 is attached to the third tube 5 near the exhalation port 2b, the exhalation assistance device 10 can more sensitively detect the pressure inside each tube, allowing accurate monitoring of the respiratory state of the patient 51. Additionally, since the flow sensor 23 is attached to the first tube 3 on the downstream side of the biasing means 11, the respiratory state of the patient 51 can also be accurately monitored by the flow sensor 23.

Since the exhalation assistance device 10 can be used by being attached to the artificial respiration device 1, such as a ventilator or anesthesia machine, the functions of the artificial respiration device 1, such as mechanical ventilation such as pressure-controlled ventilation, volume-controlled ventilation, or pressure support ventilation, manual ventilation using a breathing bag, measurement of ventilation volume, and recirculation of anesthesia gases, can be used as they are. Therefore, medical professionals do not need to become accustomed to a new artificial respiration device 1. Additionally, compared to purchasing a new device to control the entire respiratory process, the exhalation assistance device can be introduced at a lower cost. Moreover, since the exhalation assistance device can be retrofitted to the artificial respiration device 1 immediately, it is suitable for emergency use and provides reassurance as it can be removed to restore the original state.

Furthermore, the exhalation assistance device 10 can assist with exhalation in synchronization not only during artificial respiration but also during spontaneous breathing, allowing the patient 51 to inhale when they desire to inhale. Since the exhalation assistance device only assists with exhalation, it does not cause discomfort to the patient 51, and the patient 51 can exhale when they desire to exhale. Additionally, by toggling the centrifugal pump 21 on and off, the exhalation assistance device 10 can confirm whether ventilation due to the breathing of the patient 51 has improved. Moreover, it may be possible to detect severe COPD patients by using the exhalation assistance device for testing.

As shown in FIG. 1, the exhalation assistance device 10 may include an intubation tube 31 provided to be attachable to one end of the first tube 3 to communicate with the patient-side flow path. The intubation tube 31 is configured to be capable of tracheal intubation in the patient 51 to supply inspiratory air to the patient 51 and discharge expiratory air from the patient 51. Since the biasing means 11 can assist with the discharge of expiratory air, respiration can be ensured even when using an intubation tube 31 with a small diameter. Therefore, the outer diameter of the intubation tube 31 can be reduced to 7 mm or less (approximately 5 mm or less in inner diameter). This can reduce throat pain in the patient 51 during intubation and facilitate intubation in patients 51 with upper airway stenosis. Additionally, it can assist non-invasive artificial respiration without intubation of the patient 51.

Furthermore, the exhalation assistance device 10 may include a patient-side tube to which the biasing means 11 is attached, a branching joint attached to the other end of the patient-side tube, and an inhalation-side tube and an exhalation-side tube connected to the branching joint, and the branching joint may be provided to branch from the patient-side tube into the inhalation-side tube and the exhalation-side tube. In this case, a commercially available tube used by being connected to the artificial respiration device 1 can be removed and replaced entirely with the exhalation assistance device 10. Additionally, the intubation tube 31 may be attachable to one end of the patient-side tube. It is preferable that all flow paths through which the expiratory air or inspiratory air of the patient 51 passes, from the patient-side tube to the inhalation-side tube and the exhalation-side tube via the branching joint, are configured to be airtight. When the exhalation assistance device 10 is attached to the artificial respiration device 1, the patient-side tube constitutes the patient-side flow path, the inhalation-side tube constitutes the inhalation-side flow path, and the exhalation-side tube constitutes the exhalation-side flow path.

### [Example 1]

A model experiment was conducted using the exhalation assistance device 10 shown in FIG. 1. The exhalation assistance device 10 and the test lung 6 used in the experiment are shown in FIG. 2. The test lung 6 used was "SelfTestLung" manufactured by Drager. Additionally, a commercially available ventilation monitor ("GF-220R" manufactured by Nihon Kohden Corporation) was incorporated on the patient 51 side of the biasing means 11 to evaluate the performance of the exhalation assistance device 10. In the experiment, assuming a COPD patient 51, the test lung 6 was adjusted so that exhalation takes time and inhalation cannot be completed within the respiratory cycle. The respiratory rate was set to 12 breaths per minute, and the operating time of the centrifugal pump 21 was set to 0.5 seconds. The sampling frequency of the ventilation monitor data was set to 125 Hz.

The negative pressure performance of the centrifugal pump 21 used in the experiment is shown in FIG. 3. FIG. 3(a) is a graph showing the negative pressure fluctuations when the centrifugal pump 21 is operated for a sufficiently long time (approximately 9 seconds), and FIG. 3(b) is a graph showing the negative pressure fluctuations when the centrifugal pump 21 is operated for 0.5 seconds. The negative pressure measurement was performed by connecting an intubation tube with an inner diameter of 6.5 mm, the centrifugal pump 21, and the ventilation monitor ("GF-220R" manufactured by Nihon Kohden Corporation) in series, with both ends open to atmospheric pressure. As shown in FIG. 3, it was confirmed that the centrifugal pump 21 used reaches a steady state in about 2 seconds from the start of operation, but in the experiment, it stops before reaching the steady state.

The experiment was conducted by attaching the exhalation assistance device 10 to the second tube 4 and the third tube 5 connected to the artificial respiration device 1, and connecting one end of the first tube 3 to the test lung 6. FIG. 4 shows the experimental setup. During the experiment, the control unit 13 was connected to a laptop computer 7 to monitor the operating state of the centrifugal pump 21 and the respiratory state.

The fluctuations in flow rate during respiration displayed on the monitor of the artificial respiration device 1 are shown in FIG. 5. In the graph shown in FIG. 5, the positive side (upper side) of the vertical axis represents the flow rate of inspiratory air flowing from the artificial respiration device 1 to the test lung 6, and the negative side (lower side) represents the flow rate of expiratory air flowing from the test lung 6 to the artificial respiration device 1. Additionally, in the graph shown in FIG. 5, the flow rate fluctuations for the first four breaths represent the case when the exhalation assistance device 10 is not operated, and the flow rate fluctuations for the fifth and subsequent breaths (after the "assistance start" arrow in the figure) represent the case when the exhalation assistance device 10 is operated.

As shown in FIG. 5, it was confirmed that, when the exhalation assistance device 10 is not operated, the flow rate of expiratory air does not return to zero during the transition from exhalation to inhalation. However, it was confirmed that, when the exhalation assistance device 10 is operated, the flow rate of expiratory air returns to zero during the transition from exhalation to inhalation.

### [Example 2]

To investigate the flow rate fluctuations caused by the exhalation assistance device 10 in detail, an experiment was conducted with the same configuration as in Example 1, except that the test lung 6 was changed to a high-performance test lung, "ASL5000" manufactured by IngMar Medical. The experiment was conducted for the cases when the exhalation assistance device 10 was not operated (no support), when the exhalation assistance device 10 was operated with the operating time of the centrifugal pump 21 set to 0.4 seconds (weak support), and when the exhalation assistance device 10 was operated with the operating time of the centrifugal pump 21 set to 0.5 seconds (strong support). The pieces of flow rate fluctuation data for one respiratory cycle obtained in each case are superimposed and shown in FIG. 6.

As shown in FIG. 6, in the case of no support, it was confirmed that, when the transition from inhalation to exhalation occurs, the expiratory air flow rate momentarily peaks and then gradually decreases. In contrast, in the cases of weak support and strong support, it was confirmed that, when the transition from inhalation to exhalation occurs, the expiratory air flow rate momentarily peaks, and after a slight time lag, the expiratory air flow rate begins to gradually increase again, reaches another peak, and then gradually decreases. In the experiment, since the centrifugal pump 21 is set to start operating upon detecting a decrease in the pressure within the flow path after the momentary peak in exhalation, it is considered that the influence of the exhalation assistance by the centrifugal pump 21 causes the expiratory air flow rate to gradually increase again. Additionally, it is considered that while the centrifugal pump 21 is operating or rotating due to inertia after stopping, the expiratory air flow rate gradually increases due to its influence.

As shown in FIG. 6, it was confirmed that, in the case of no support, the flow rate of expiratory air does not return to zero during the transition from exhalation to inhalation, and in the case of weak support, the flow rate of expiratory air is slightly above zero during the transition from exhalation to inhalation. In contrast, in the case of strong support, it was confirmed that the flow rate of expiratory air returns to zero during the transition from exhalation to inhalation. From these results, it can be said that the exhalation assistance device 10 can assist with the discharge of the expiratory air from the patient 51. Additionally, it can be said that appropriate exhalation assistance can be achieved by adjusting the operating time or rotational speed of the centrifugal pump 21 to control the discharge amount of expiratory air.

### [Example 3]

An animal experiment using pigs was conducted with the exhalation assistance device 10 shown in FIG. 1. In the experiment, an intubation tube was inserted into the trachea of an anesthetized pig, one end of the first tube 3 was connected to the intubation tube, and artificial respiration was performed using the artificial respiration device 1. The respiratory rate was set to 15 breaths per minute, and the operating time of the centrifugal pump 21 was set to 0.5 seconds. Other experimental conditions were the same as in Example 2. The pieces of pressure fluctuation data for one respiratory cycle measured by the ventilation monitor when the exhalation assistance device 10 was not operated (no support) and when it was operated (with support) are superimposed and shown in FIG. 7(a), and the pieces of flow rate fluctuation data measured by the ventilation monitor are superimposed and shown in FIG. 7(b).

As shown in FIGS. 7(a) and 7(b), it was confirmed that, when the exhalation assistance device 10 was not operated, during the transition from inhalation to exhalation, the pressure in the flow path significantly decreases, the exhalation flow rate peaks, and then the pressure and flow rate gradually decrease. In contrast, it was confirmed that, when the exhalation assistance device 10 was operated, during the transition from inhalation to exhalation, the pressure in the flow path significantly decreases, and the exhalation flow rate peaks. Then, due to the influence of exhalation assistance by the centrifugal pump 21, after a slight time lag, the pressure begins to gradually decrease again, the exhalation flow rate begins to increase, and after another peak in pressure decrease and exhalation flow rate, the pressure gradually increases while the exhalation flow rate decreases. Additionally, it was confirmed that, when the exhalation assistance device 10 was operated, the pressure in the patient-side flow path became negative, indicating that a significant negative pressure was applied to the patient-side flow path.

As shown in FIGS. 7(a) and 7(b), by applying negative pressure to the patient-side flow path with the centrifugal pump 21 during exhalation, the flow rate on the exhalation side increases, indicating that exhalation assistance is achieved in the animal experiment as well.

### [Example 4]

An animal experiment was conducted using six pigs under the same conditions as in Example 3 with the exhalation assistance device 10 shown in FIG. 1. The pieces of flow rate fluctuation data for three consecutive respiratory cycles measured by the ventilation monitor when the exhalation assistance device 10 was not operated (no support) and when it was operated (with support) are superimposed for the six pigs and shown in FIG. 8(a), and the pieces of circuit internal pressure fluctuation data are superimposed for the six pigs and shown in FIG. 8(b). Additionally, for the cases of no support and with support, curves (PV loops) connecting the relationship between circuit internal pressure and ventilation volume ratio (the ratio of ventilation volume when the maximum ventilation volume during non-operation of the exhalation assistance device 10 is set to 1) for a representative respiratory cycle are superimposed for the six pigs and shown in FIG. 8(c).

As shown in FIGS. 8(a) and 8(b), it was confirmed that all six pigs exhibited the same trends in circuit internal pressure and flow rate as shown in FIGS. 7(a) and 7(b). Additionally, as shown in FIG. 8(c), it was confirmed that, when support was provided, compared to no support, the lung internal pressure during exhalation decreased, and the ventilation volume during inhalation increased by approximately 20%. From these results, it can be said that the exhalation assistance device 10 can assist with the discharge of expiratory air even with individual differences.

### [Example 5]

An animal experiment was conducted using a pig under the same conditions as in Example 3 with the exhalation assistance device 10 shown in FIG. 1. In the experiment, the pig was equipped with EIT (Electrical Impedance Tomography; "EIT system Enlight 2100" manufactured by TIMPEL S.A.), and total impedance changes were also measured. The temporal changes in circuit internal pressure measured by the ventilation monitor before and after operating the exhalation assistance device 10 are shown in FIG. 9(a), and the temporal changes in ventilation volume are shown in FIG. 9(b). Additionally, the temporal changes in total impedance measured by EIT are shown in FIG. 9(c). In each figure, the timing of starting the operation of the exhalation assistance device 10 is indicated by a downward arrow.

As shown in FIGS. 9(a) to 9(c), it was confirmed that the circuit internal pressure during exhalation decreased, and the ventilation volume increased by approximately 20% with the operation of the exhalation assistance device 10. Additionally, it was confirmed that the total impedance during exhalation changed to become smaller. This indicates a decrease in lung volume during exhalation, which is considered to be due to the promotion of exhalation by the exhalation assistance device 10.

### [Example 6]

An animal experiment was conducted using a pig with the exhalation assistance device 10 shown in FIG. 1. In the experiment, the pig was equipped with EIT, and while artificial respiration was performed on the pig using the artificial respiration device 1, measurements of circuit internal pressure and ventilation volume by the ventilation monitor and total impedance changes by EIT were conducted. The pressure-controlled ventilation settings of the artificial respiration device 1 were fixed at an inhalation time of 1.35 seconds, an inspiratory pressure (driving pressure) of 15 cmH₂O, and a positive end-expiratory pressure (PEEP) of 5 cmH₂O, and measurements were conducted by varying the respiratory rate (RR) and the on/off state of the exhalation assistance device 10 as follows: 15 breaths (off) → 20 breaths (off) → 25 breaths (off) → 25 breaths (on) → 20 breaths (on) → 15 breaths (on) → 15 breaths (off).

The temporal changes in circuit internal pressure measured by the ventilation monitor during artificial respiration are shown in FIG. 10(a), and the temporal changes in ventilation volume are shown in FIG. 10(b). Additionally, the temporal changes in total impedance measured by EIT are shown in FIG. 10(c).

As shown in FIGS. 10(a) to 10(c), it was confirmed that, when the exhalation assistance device 10 was not operated, as the respiratory rate increased (i.e., exhalation time shortened) (from 0 to approximately 215 seconds), the circuit internal pressure at the end of exhalation increased, the one-time ventilation volume decreased, and the total impedance increased. This is considered to be due to the inability to complete exhalation within the exhalation time as the exhalation time shortens, resulting in lung overexpansion. In contrast, it was confirmed that, when the exhalation assistance device 10 was operated (from approximately 215 to approximately 360 seconds), the circuit internal pressure at the end of exhalation decreased, the one-time ventilation volume increased, and the total impedance decreased. This is considered to be due to the promotion of exhalation and suppression of lung overexpansion with the operation of the exhalation assistance device 10.

### [Example 7]

An animal experiment was conducted using a pig with the exhalation assistance device 10 shown in FIG. 1. In the experiment, the pig was equipped with EIT, and while artificial respiration was performed on the pig using the artificial respiration device 1, measurements of circuit internal pressure and ventilation volume by the ventilation monitor and total impedance changes by EIT were conducted. The pressure-controlled ventilation settings of the artificial respiration device 1 were fixed at an inhalation time of 1.35 seconds and a respiratory rate of 15 breaths, and measurements were conducted by varying the inspiratory pressure (driving pressure Δ), positive end-expiratory pressure (PEEP), and the on/off state of the exhalation assistance device as follows: PEEP 5 Δ15 (off → on) → PEEP 0 (=ZEEP) Δ15 (off → on) → ZEEP Δ20 (off → on). The units of driving pressure Δ and positive end-expiratory pressure PEEP are cmH₂O.

The temporal changes in circuit internal pressure measured by the ventilation monitor during artificial respiration are shown in FIG. 11(a), and the temporal changes in ventilation volume are shown in FIG. 11(b). Additionally, the temporal changes in total impedance measured by EIT are shown in FIG. 11(c).

As shown in FIGS. 11(a) to 11(c), it was confirmed that, when the exhalation assistance device 10 was operated, in any artificial respiration setting, the circuit internal pressure decreased, the ventilation volume increased, and the total impedance decreased. This is considered to be due to the promotion of exhalation and reduction in lung volume with the operation of the exhalation assistance device 10, even when PEEP is 0 cmH₂O (=ZEEP).

### [Example 8]

An animal experiment was conducted using a pig under the same conditions as in Example 3 with the exhalation assistance device 10 shown in FIG. 1. In the experiment, while artificial respiration was performed on the pig using the artificial respiration device 1, measurements of biological information (pulse rate (PR), percutaneous arterial blood oxygen saturation (SpO₂), pulmonary artery pressure (PAP) (systolic: sys, diastolic: dias), arterial pressure (ART) (systolic: sys, diastolic: dias), central venous pressure (CVP), continuous cardiac output (CCO), and end-tidal carbon dioxide partial pressure (EtCO₂)) and blood gas analysis (arterial oxygen partial pressure (PaO₂) and arterial carbon dioxide partial pressure (PaCO₂)) were conducted before and after operating the exhalation assistance device 10. The measurement results are shown in FIGS. 12(a) and 12(b). In each figure, the timing of starting the operation of the exhalation assistance device 10 is indicated by a downward arrow.

As shown in FIG. 12(a), it was confirmed that the pulse rate (PR) increased with the operation of the exhalation assistance device 10. Additionally, as shown in FIG. 12(b), it was confirmed that after the operation of the exhalation assistance device 10, the end-tidal carbon dioxide partial pressure (EtCO₂) and arterial carbon dioxide partial pressure (PaCO₂) decreased, and the arterial oxygen partial pressure (PaO₂) increased. No significant changes were observed in other biological information.

### Reference Signs List

1 Artificial respiration device
1a
2 Device body
2a Inhalation port
2b Exhalation port
3 First tube
4 Second tube
5 Third tube
6 Test lung
7 Laptop computer
10 Exhalation assistance device
11 Biasing means
21 Centrifugal pump
12 Sensor
22 Pressure sensor
23 Flow sensor
13 Control unit
24 Microcomputer
25 Relay circuit
31 Intubation tube
51 Patient

## Claims

1. An exhalation assistance device used by being attached to an artificial respiration device to assist the discharge of expiratory air from a patient, the artificial respiration device having a patient-side flow path provided such that one end side can be attached to the patient, and an inhalation-side flow path and an exhalation-side flow path communicating with the other end of the patient-side flow path, the artificial respiration device configured to supply inspiratory air to the patient from the inhalation-side flow path through the patient-side flow path to inflate the patient's lungs during inhalation, and to discharge expiratory air from the patient from the patient-side flow path through the exhalation-side flow path during exhalation, the exhalation assistance device comprising:
a biasing means provided to be attachable to the patient-side flow path and configured to, when attached to the patient-side flow path, allow the inspiratory air supplied to the patient to pass through while biasing expiratory air from the patient in a discharge direction;
a sensor provided to be capable of measuring a pressure in at least one of the patient-side flow path, the inhalation-side flow path, or the exhalation-side flow path, and/or a flow rate of the inspiratory and expiratory air of the patient; and
a control unit connected to the biasing means and the sensor and provided to be capable of drive controlling the biasing means during exhalation based on measurement data from the sensor.

2. The exhalation assistance device according to claim 1, wherein the sensor includes a pressure sensor provided to measure the pressure in at least one of the patient-side flow path, the inhalation-side flow path, or the exhalation-side flow path.

3. The exhalation assistance device according to claim 1, wherein the sensor includes a flow sensor provided in the patient-side flow path to measure the flow rate of the inspiratory and expiratory air of the patient.

4. The exhalation assistance device according to claim 1, comprising:
a patient-side tube provided to be capable of constituting the patient-side flow path, to which the biasing means is attached;
a branching joint attached to the other end of the patient-side tube so that the patient-side flow path branches into the inhalation-side flow path and the exhalation-side flow path on the other end side of the patient-side tube;
an inhalation-side tube connected to the branching joint to be able to constitute the inhalation-side flow path; and
an exhalation-side tube connected to the branching joint to be able to constitute the exhalation-side flow path.

5. The exhalation assistance device according to claim 4, comprising an intubation tube provided to be attachable to one end of the patient-side tube to communicate with the patient-side flow path,
wherein the intubation tube is configured to be capable of tracheal intubation in the patient to supply inspiratory air to the patient and discharge the expiratory air from the patient.

6. The exhalation assistance device according to claim 5, wherein the intubation tube has an outer diameter of 7 mm or less.

7. An artificial respiration device comprising the exhalation assistance device according to any one of claims 1 to 6.
